(19) Europäisches Patentamt
European Patent Office
Office européen des brevets

(11) Veröffentlichungsnummer : **0 178 524 B2**

(12) # NEUE EUROPÄISCHE PATENTSCHRIFT

(45) Veröffentlichungstag der neuen Patentschrift : **12.01.94 Patentblatt 94/02**

(51) Int. Cl.$^5$ : **C07C 57/42,** C07C 51/00, B01J 23/06, B01J 23/74

(21) Anmeldenummer : **85112383.6**

(22) Anmeldetag : **01.10.85**

(54) **Verfahren zur Herstellung von gegebenenfalls substituierter Zimtsäure in Gegenwart eines Katalysators.**

(30) Priorität : **13.10.84 DE 3437634**

(43) Veröffentlichungstag der Anmeldung : **23.04.86 Patentblatt 86/17**

(45) Bekanntmachung des Hinweises auf die Patenterteilung : **01.06.88 Patentblatt 88/22**

(45) Bekanntmachung des Hinweises auf die Entscheidung über den Einspruch : **12.01.94 Patentblatt 94/02**

(84) Benannte Vertragsstaaten : **CH DE FR GB IT LI NL**

(56) Entgegenhaltungen : **US-A- 3 766 259**

(56) Entgegenhaltungen : **US-A-24 464 20**
**Kirk-Othmer,Encyclopedia of Chemical Technology,Ed. 3, vol. 13, (1981), 764-765, vol. 14 (1981), 616**

(73) Patentinhaber : **BAYER AG**
**D-51368 Leverkusen (DE)**

(72) Erfinder : **Dorlars, Alfons, Dr.**
**Mozartstr. 32**
**D-5090 Leverkusen 1 (DE)**
Erfinder : **Blank, Heinz Ulrich, Dr.**
**Am Geusfelde 35**
**D-5068 Odenthal (DE)**
Erfinder : **Nordt, Herbert, Dr.**
**verstorben (DE)**
Erfinder : **Trescher, Viktor, Dr.**
**Völklinger Str. 7**
**D-5090 Leverkusen (DE)**

**EP 0 178 524 B2**

## Beschreibung

Die Erfindung betrifft ein Verfahren zur katalytischen Herstellung von gegebenenfalls substituierter Zimtsäure aus dem entsprechenden Benzaldehyd und Keten in Gegenwart eines Katalysators aus der Gruppe der Eisen- und/oder Zinksalze von $C_2$-$C_{20}$-Carbonsäuren, der Anteile der entsprechenden freien Carbonsäuren enthält.

Zimtsäure wird im allgemeinen technisch nach dem sogenannten Perkin-Verfahren [Houben-Weyl, 4. Auflage, Band 8, Seite 442 ff. (1952); Org. React. I, Seite 210 bis 265 (1942); Ullmanns Encyclopädie der technischen Chemie, 3. Auflage, Band 19, Seite 36 (1969)] aus dem entsprechenden Benzaldehyd, Acetanhydrid und einem alkalischen Kondensationsmittel hergestellt.

Nachteilig aus dem Perkin-Verfahren sind die Verarbeitung von grossen Mengen an Alkaliacetaten, grossen Überschüssen an Acetanhydrid und lange Reaktionszeiten.

Es ist auch bekannt, Benzaldehyd und Keten in Gegenwart von Kaliumacetat zu einem Primärprodukt umzusetzen, aus dem man etwa 22% Zimtsäure erhält [J. Am. Chem. Soc. 55, 275 (1933)]. Unter ähnlichen Bedingungen wurde auch 30% Zimtsäure und 70% Styrol erhalten [Organic Synthesis, Vol. I, 215 und 216 C.D. Hurd. C.L. Thomas, (1942)].

Aus Ind. Eng. chem. 41, 768 (1949) ist die Umsetzung von Benzaldehyd mit Keten bei 60°C unter Zusatz von Natriumacetat und anschliessendem Erhitzen des Reaktionsproduktes auf Siedetemperatur bekannt. Man erhält dabei Zimtsäure mit einer Ausbeute von 40%.

Aus der US-PS 2 484 067 (1969) ist bekannt, als Katalysator für diese Umsetzung Bortrifluorid zu verwenden. Man erhält dabei Zimtsäure mit einer Ausbeute von 42%.

Bei den bekannten Umsetzungen von Benzaldehyd mit Keten in Gegenwart eines Katalysators entstehen Produkte, die hohe Styrolanteile aufweisen.

Es wurde ein Verfahren zur Herstellung von gegebenenfalls substituierten Zimtsäuren durch Umsetzung des entsprechenden Benzaldehyds mit Keten in Gegenwart eines Katalysators gefunden, das dadurch gekennzeichnet ist, dass man einen Katalysator aus der Gruppe der Eisen- und/oder Zinksalze einer Mono- oder Dicarbonsäure mit 2 bis 20 Kohlenstoffatomen, wobei die einzusetzenden Katalysatoren einen Anteil der entsprechenden freien Carbonsäuren von 1 - 80 Gew.-%, bezogen auf den gesamten Katalysator enthalten, verwendet und die Umsetzung in einem Lösungsmittel durchführt und dann das Reaktionsprodukt in Gegenwart eines sauren oder basischen Katalysators im Temperaturbereich von 100 bis 250°C behandelt.

Unter Anwendung solcher Katalysatoren gelingt es, gegebenenfalls substituierte Zimtsäuren mit hohen Ausbeuten herzustellen.

Die erfindungsgemäss einzusetzenden Eisen- und Zinksalze sind bevorzugt solche von Mono- oder Dicarbonsäuren der Formel

$$X - A - COOH$$

in der

X Carboxyl oder Wasserstoff bedeutet, und

A einen zweibindigen Rest, der sich aus der Gruppe gesättigter oder ungesättigter aliphatischer Kohlenwasserstoffe mit 1 bis 18 Kohlenstoffatomen, cycloaliphatischer Kohlenwasserstoffe mit 5 bis 12 Kohlenstoffatomen, araliphatischer Kohlenwasserstoffe mit 7 bis 18 Kohlenstoffatomen oder aromatischer Kohlenwasserstoffe mit 6 bis 1 2 Kohlenstoffatomen ableitet und für den Fall, dass X für eine Carboxylgruppe steht, auch eine Einfachbindung bedeuten kann.

Gesättigte aliphatische Kohlenwasserstoffe, von denen sich die zweibindigen Reste ableiten, sind hierbei im allgemeinen geradkettige oder verzweigte Kohlenwasserstoffe mit 1 bis 18, bevorzugt 1 bis 12 Kohlenstoffatomen. Beispielsweise seien die folgenden gesättigten aliphatischen Kohlenwasserstoffe genannt: Methan, Ethan, Propan, Butan, Isobutan, Pentan, Isopentan, Hexan, Isohexan, Heptan, Isoheptan, Octan, Isooctan, Nonan, Isononan, Decan, Isodecan, Undecan, Isoundecan, Dodecan und Isododecane.

Ungesättigte aliphatische Kohlenwasserstoffe, von denen sich die zweibindigen Reste ableiten, sind hierbei im allgemeinen geradkettige oder verzweigte Kohlenwasserstoffe mit 2 bis 18, bevorzugt 2 bis 12, Kohlenstoffatomen und einer oder zwei, bevorzugt einer, Doppelbindung. Beispielsweise seien die folgenden ungesättigten aliphatischen Kohlenwasserstoffe genannt: Ethen, Propen, Buten, Heptadecen, tert.-Butylen und Neopentylen.

Cycloaliphatische Reste sind hierbei im allgemeinen cyclische Kohlenwasserstoffreste mit 5 bis 12, bevorzugt 5 bis 8, Kohlenstoffatomen. Beispielsweise seien die folgenden cycloaliphatischen Reste genannt: Cyclopentylen, Cyclohexylen, Cycloheptylen und Cyclooctylen.

Araliphatische Reste sind hierbei im allgemeinen aus einem Alkylenteil und einem aromatischen Teil bestehende Reste. Der Alkylenteil kann hierbei ein geradkettiger oder verzweigter, gesättigter oder ungesättigter Kohlenwasserstoffrest mit 1 bis 12, bevorzugt 1 bis 6, Kohlenstoffatomen und gegebenenfalls einer oder zwei,

bevorzugt einer Doppelbindung sein. Der aromatische Teil kann hierbei beispielsweise ein Rest aus der Benzolreihe mit 6 bis 12 Kohlenstoffatomen sein. Beispielsweise seien die folgenden araliphatischen Reste genannt: Benzylen, Xylylen.

Aromatische Reste sind hierbei im allgemeinen Re ste aus der Benzolreihe mit 6 bis 12 Kohlenstoffatomen. Beispielsweise seien die folgende Reste genannt: Phenylen, Naphthylen und Biphenylen, Toluylen und Xylylen. Bevorzugt ist der Phenylenrest.

Die erfindungsgemäss einzusetzenden Eisen- und/oder Zinksalze sind insbesondere bevorzugt solche von Monocarbonsäuren der Formel

H - B - COOH

in der

B einen zweibindigen Rest aus der Gruppe der gesättigten aliphatischen Reste mit 1 bis 12 Kohlenstoffatomen, cycloaliphatische Reste mit 5 oder 6 Kohlenstoffatomen, araliphatische Reste mit 7 bis 9 Kohlenstoffatomen oder aromatische Reste mit 6 bis 10 Kohlenstoffatomen bedeuten kann. Beispielsweise seien die Eisen- und/oder Zinksalze der folgenden Mono- oder Dicarbonsäuren genannt: Essigsäure, Propionsäure, Buttersäure, Isobutter- säure. Valeriansäure, Isovaleriansäure, Pivalinsäure, Ethyl-methyl-essigsäure, 2-Ethylbutansäure, Dodecansäure, Stearinsäure, Hexahydrobenzoesäure, Benzoesäure, Phenylessigsäure, Zimtsäure, Oxalsäure, Malonsäure, Bernsteinsäure, Adipinsäure und Phthalsäure.

Erfindungsgemäss können als Katalysatoren die Eisen- oder Zinksalze verwendet werden. Es ist selbstverständlich auch möglich, Gemische der Eisen- und Zinksalze zu verwenden.

In den erfindungsgemäss einzusetzenden Katalysatoren enthalten die Eisen- und/oder Zinksalze Anteile der entsprechenden freien Carbonsäuren in einer Menge von 1 - 80 Gew.-% der freien Carbonsäure. Bevorzugt enthalten die Katalysatoren 1 bis 50 Gew.-% der freien Carbonsauren, bezogen auf den gesamten Katalysator.

Die Katalysatoren können hergestellt werden, indem man den neutralen oder basischen Salzen, bevorzugt den neutralen Salzen, des Zinks oder des 2- oder 3wertigen Eisens die entsprechende Menge der Carbonsäure zusetzt.

Selbstverständlich kann man auch Eisen- und/oder Zinksalze der Mono- oder Dicarbonsäuren verwenden, die noch einen Säureanteil aus ihrem Herstellungsverfahren enthalten. Die Eisen- und Zinksalze werden im allgemeinen aus den entsprechenden Oxiden oder Carbonaten und den entsprechenden Carbonsäuren hergestellt.

Schliesst man bei der Umsetzung Carbonsäuren aus, so kommt die Reaktion praktisch nicht in Gang oder verläuft unbefriedigend.

Das erfindungsgemässe Verfahren kann durch die folgende Reaktionsgleichung erläutert werden:

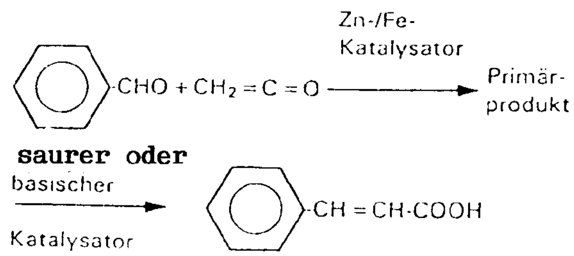


Bei der Umsetzung des gegebenenfalls substituierten Benzaldehyds mit Keten in Gegenwart des erfindungsgemässen Katalysators entsteht ein Primärprodukt, aus dem in erfindungsgemässer Weise der Zimtaldehyd hergestellt wird.

Bei dem Primärprodukt handelt es sich im allgemeinen im wesentlichen um oligomere Polyester der β-Hydroxy-β-phenyl-propionsäure mit terminalen Acetyl- bzw. Acetoxygruppen und einem mittleren Molekulargewicht von 1000 bis 1 500, die als helles, zähviskoses Produkt isoliert werden können.

Bei negativ substituierten Benzaldehyden ist es jedoch auch möglich, dass die Primärprodukte als β-Aryl-β-propiolactone vorliegen können.

Für das erfindungsgemässe Verfahren ist es jedoch nicht erforderlich, das Primärprodukt zu isolieren. Die Umsetzung des gegebenenfalls substituierten Benzaldehyds mit Keten und die nachfolgende Umsetzung des Primärprodukts werden in der Regel als Eintopfreaktion durchgeführt.

Gegebenenfalls substituierte Benzaldehyde für das erfindungsgemässe Verfahren können alle, aufgrund ihrer Reaktivität möglichen, Benzaldehyde sein.

Bevorzugte Benzaldehyde für das erfindungsgemässe Verfahren sind Verbindungen der Formel

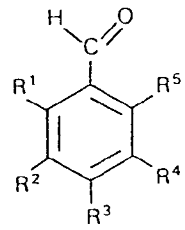

in der vorzugsweise eine ortho-Stellung unsubstituiert ist und

$R^1$ bis $R^5$ gleich oder verschieden sind und Wasserstoff, Halogen, Hydroxy, Nitro, Niederalkyl, Niederalkoxy oder gegebenenfalls substituiertes Phenyl bedeuten und wobei an zwei benachbarten Resten ein aromatischer Ring ankondensiert sein kann oder wobei zwei benachbarte Reste durch die Gruppe

$$-O-CH_2-O-$$

verbunden sein können.

Halogen bedeutet hierbei im allgemeinen Fluor, Chlor, Brom oder Iod, bevorzugt Chlor oder Brom.

Niederalkyl bedeutet beispielsweise einen geradkettigen oder verzweigten Kohlenwasserstoffrest mit 1 bis 6 Kohlenstoffatomen. Beispielsweise seien die folgenden Niederalkylreste genannt: Methyl, Ethyl, Propyl, Isopropyl, Butyl, Isobutyl, Pentyl, Isopentyl, Hexyl und Isohexyl.

Niederalkoxy bedeutet beispielsweise einen Alkyletherrest mit 1 bis 6 Kohlenstoffatomen. Beispielsweise seien die folgenden Niederalkoxyreste genannt: Methoxy, Ethoxy, Propoxy, Isopropoxy, Butoxy, Isobutoxy, Pentoxy, Isopentoxy, Hexoxy und Isohexoxy. Bevorzugt sind der Methoxy- und Ethoxyrest.

Der Phenylrest kann gegebenenfalls substituiert sein. Als Substituenten seien beispielsweise genannt: Niederalkyl mit bis etwa 6 C-Atomen, Niederalkoxy mit bis etwa 6 C-Atomen, Halogen.

Für den Fall, dass an zwei benachbarten Resten $R^1$ bis $R_5$ ein aromatischer Ring ankondensiert ist, liegt beispielsweise ein Naphthalinsystem vor.

Für den Fall, dass zwei benachbarte Reste $R_1$ bis $R_5$ durch die Gruppe $-O-CH_2-O-$ verbunden sind, liegt beispielsweise ein Methylen-dioxy-phenyl-System vor.

Besonders bevorzugt sind Benzaldehyde der Formel

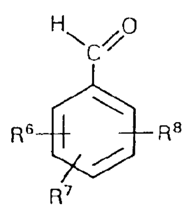

in der vorzugsweise eine ortho-Stellung unsubstituiert ist und

$R^6$ bis $R^8$ gleich oder verschieden sind und Wasserstoff, Fluor, Chlor, Brom, Nitro, Methyl, Ethyl, Methoxy, Ethoxy oder Phenyl bedeuten und wobei zwei benachbarte Reste durch die Gruppe

$$-O-CH_2-O-$$

verbunden sein können.

Beispielsweise seien die folgenden gegebenenfalls substituierten Benzaldehyde genannt: Benzaldehyd, o-, m- und p-Fluorbenzaldehyd, o-, m- und p-Chlorbenzaldehyd, o-, m- und p-Brombenzaldehyd, o-, mund p-Iodbenzaldehyd, 3-Brom-4-fluorbenzaldehyd, 2-Chlor-6-fluorbenzaldehyd, 2,4-, 2,5-, 2,6- und 3,5-Dichlorbenzaldehyd, 2,3,4-, 2,4,5-, 2,3,6- und 3,4,5-Trichlorbenzaldehyd, Pentachlorbenzaldehyd, o-, m- und p-Tolylaldehyd, 3- und 4-Ethylbenzaldehyd, 4-Isopropyl- und 4-Phenylbenzaldehyd, 4-Butylbenzaldehyd, 2-, 3- und 4-Methoxybenzaldehyd, 4-Ethoxy-, 4-Propoxy-, 4-Butoxy- und 4-Phenoxy-benzaldehyd, Piperonal, 2,4-Dimethoxy- und 3,4-Dimethoxybenzaldehyd, o-, m- und p-Nitrobenzaldehyd, 4-Acetoxybenzaldehyd- 4-Hydroxy-benzaldehyd, 2-Nitro-4-chlorbenzaldehyd, 2-Nitro-4-brombenzaldehyd, 2,4- und 2,6-Dinitrobenzaldehyd, 4-Methoxy-3-nitrobenzaldehyd, 4-Methyl-2-fluor-benzaldehyd, 4-Methyl-2,3-dichlorbenzaldehyd, 4-Methyl-3-nitro benzaldehyd, 2,4,6-Trimethyl-3,5-dinitrobenzaldehyd, 4'-Chlor-4-formylbiphenyl, 3-Methoxy-2-, 3-Methoxy-4- und 3-Methoxy-6-nitro-benzaldehyd, 3,4,5-Trimethylbenzaldehyd, Naphthaldehyd-1, Naphthaldehyd-2 und 4-Chlornaphthaldehyd.

Im besonderen bevorzugt ist die Umsetzung von Benzaldehyd.

Die gegebenenfalls substituierten Benzaldehyde für das erfindungsgemässe Verfahren sind bekannt und können nach bekannten Verfahren hergestellt werden.

Als Keten für das erfindungsgemässe Verfahren wird im allgemeinen gasförmiges Keten verwendet, das beispielsweise durch pyrogene Spaltung von Aceton oder Essigsäure an einer Chrom-Nickel-Spirale hergestellt werden kann (Houben-Weyl, Band VII/4. Seite 68), wobei auch technische, gegebenenfalls verunreinigte Qualitäten verwendet werden können.

Für das erfindungsgemässe Verfahren setzt man im allgemeinen 0,40 bis 1,20 Mol, bevorzugt 0,50 bis 0,96 Mol Keten, bezogen auf 1 Mol des gegebenenfalls substituierten Benzaldehyds, ein.

Für das erfindungsgemässe Verfahren setzt man im allgemeinen 0,1 bis 10 Mol-%, bevorzugt 0,5 bis 4 Mol-% des Katalysators aus der Gruppe der Eisen-und/oder Zinksalze, bezogen auf eingesetztes Benzaldehyd, ein.

Das erfindungsgemäße Verfahren wird mit einem Lösungsmittel durchgeführt.

Als Lösungsmittel für das erfindungsgemässe Verfahren kommen solche in Betracht, die sich gegenüber den Reaktionskomponenten indifferent verhalten, d.h., die Dimerisierung des Ketens nicht fördern und bei Reaktionstemperatur flüssig sind.

Bevorzugt seien Lösungsmittel aus der Reihe der Kohlenwasserstoffe [Aliphaten ($C_6$ bis $C_{12}$), Aromaten ($C_6$ bis $C_{12}$) und Alkylaromaten ($C_7$ bis $C_{18}$)], Halogenkohlenwasserstoffe, Ether und Ester genannt, beispielsweise Petroleumfraktionen mit Siedepunkten ab 130°C, Toluol, Ethylbenzol, Cyclohexylbenzol, Xylol, Chlorbenzol, Dichlorbenzol, Trichlorbenzol, Methylnaphthaline, Biphenyl-diphenyl-ether-Mischungen, Dipropylether, Dibutylether, Ditolylether, Ethylacetat, Butylacetat und Glykolmethyletheracetat.

Soll die Nachbehandlung des Primärproduktes bei Normaldruck und ohne Zwischenisolierung ausgeführt werden, so setzt man zweckmässig Lösungsmittel mit einem Siedepunkt ein, der mindestens so hoch ist, wie die Temperatur, die bei der Nachbehandlung angewendet werden soll.

Es können auch niedriger siedende Lösungsmittel verwendet werden, wenn unter Druck gearbeitet oder das Lösungsmittel vor oder während der Nachbehandlung des Primärproduktes abdestilliert werden soll.

Ist die Isolierung der gebildeten Zimtsäure durch wässrige alkalische Extraktion beabsichtigt, so sollte das verwendete Lösungsmittel eine möglichst vollständige Mischungslücke mit wässrigen Systemen haben. Als vorteilhaft haben sich technische Isomerengemische von Xylolen oder Dichlorbenzolen er wiesen.

Die erfindungsgemässe Umsetzung von Benzaldehyd mit Keten wird im allgemeinen im Temperaturbereich von - 10 bis + 100°C, bevorzugt von + 20 bis + 70°C, durchgeführt. Im allgemeinen wird dieser Reaktionsschritt bei Normaldruck durchgeführt. Es ist jedoch auch möglich, die Umsetzung bei geringem Unter- oder Überdruck durchzuführen (beispielsweise im Druckbereich von 500 mbar bis 10 bar). Bei einem geringen Überdruck kann oft die schnelle und quantitative Ausnutzung des Ketensgefördet werden.

In einer besonderen Ausführungsform des erfindungsgemässen Verfahrens setzt man den Benzaldehyd nicht quantitativ um, sondern bevorzugt nur 65 bis 96%. Der nicht verbrauchte Anteil des Benzaldehyds kann bei der späteren Aufarbeitung quantitativ abdestilliert und einer erneuten Umsetzung zugeführt werden. Bei einer so abgestimmten Reaktionsführung ist die Ketenausnutzung praktisch quantitativ.

Das in der ersten Reaktionsstufe erhaltene Primärprodukt wird in der zweiten Reaktionsstufe thermisch gespalten.

Die Spaltung wird im allgemeinen im Temperaturbereich von 100 bis 250°C, bevorzugt von 150 bis 190°C, durchgeführt.

Im allgemeinen führt man die Spaltung bei Normaldruck durch. Es istjedoch auch möglich, die Spaltung bei Unter- oder Überdruck (beispielsweise im Druckbereich von 100 mbar bis 25 bar) durchzuführen.

Die erfindungsgemässe Nachbehandlung des Primärproduktes wird in Gegenwart eines sauren oder vorzugsweise basischen Katalysators durchgeführt.

Als saure Katalysatoren für die Spaltung können starke Säuren eingesetzt werden. Als starke Säuren seien beispielsweise genannt: Schwefelsäure, Chlorwasserstoff, Bromwasserstoff, Toluolsulfonsäure, Pyridiniumchlorid und Natriumhydrogensulfat.

Als basische Katalysatoren können beispielsweise basische Alkali- oder Erdalkaliverbindungen, Amine oder Azaverbindungen eingesetzt werden.

Basische Alkali- oder Erdalkaliverbindungen sind beispielsweise Alkali- und Erdalkalioxide und -hydroxide, wie Natrium- und Kaliumhydroxid, Natron- und Kalilauge, Calciumoxid, Calciumhydroxid, Alkalicarbonate, wie Natrium- und Kaliumcarbonat, Alkalisalze von Fettsäuren, wie Natriumacetat, Natriumbutyrat, Natriumpivalat und Alkoholate, wie Natriummethanolat und Kaliumisobutanolat.

Als Amine seien tertiäre Stickstoffbasen wie Tributylamin, Dimethylanilin, 4-Dimethylaminopyridin, genannt.

Azaverbindungen können beispielsweise Diazabicyclononen und Diazabicycloundecen sein.

Als basischer Katalysator wird bevorzugt Natriumhydroxid verwendet.

Der Katalysator für die Nachbehandlung des Primärproduktes wird im allgemeinen in einer Menge von 0,05 bis 0,20 Mol, bezogen auf 1 Mol eingesetztem, gegebenenfalls substituiertem Benzaldehyd, angewendet.

Im allgemeinen wird die Nachbehandlung des Primärproduktes in dem gleichen Medium durchgeführt, das für die Umsetzung des gegebenenfalls substituierten Benzaldehyds mit dem Keten verwandt wurde. Selbstverständlich ist es jedoch auch möglich, das in der ersten Reaktionsstufe verwandte Lösungsmittel abzutrennen und für die zweite Reaktionsstufe ein anderes Lösungsmittel zu verwenden oder ohne Lösungsmittel zu arbeiten.

Das erfindungsgemässe Verfahren kann sowohl kontinuierlich als auch diskontinuierlich durchgeführt werden.

Das erfindungsgemässe Verfahren kann beispielsweise wie folgt durchgeführt werden:

Zur Bildung des Primärproduktes aus gegebenenfalls substituiertem Benzaldehyd und Keten leitet man Ketengas unter wirksamer Verteilung in den betreffenden Benzaldehyd bzw. die betreffende Benzaldehydlösung in Gegenwart des erfindungsgemäss einzusetzenden Katalysators ein. Die Umsetzung ist beendet, wenn die erforderliche Menge Keten verbraucht ist.

Zur Durchführung der Nachbehandlung des Primärproduktes setzt man den sauren oder den basischen Katalysator zu und erhitzt auf die gewünschte Spalttemperatur.

Die Isolierung der gebildeten Zimtsäure kann nach gebräuchlichen Techniken erfolgen. Man kann das Losungsmittel abdampfen und die verbleibende rohe Zimtsäure durch Umlösen aus Wasser oder geeigneten Lösungsmitteln reinigen. Ebensogut kann die rohe Zimtsäure auch direkt weiterverarbeitet werden, z.B. zu Zimtsäureestern, die dann ihrerseits gereinigt oder in geeigneter Weise verwendet werden können. Nach dieser Verfahrensvariante können z.B. vorteilhaft hergestellt werden: Methyl-, Ethyl-, Propyl-, Isopropyl-, Butylcinnamat. Vorteilhafter ist oft eine Extraktion aus der Lösungsmittelphase mit Hilfe von verdünnten wässrigen Alkalihydroxiden oder Ammoniak. Aus der abgetrennten wässrigen, Alkalicinnamat bzw. Ammoniumcinnamat enthaltenden Phase lässt sich, gegebenenfalls nach Passieren eines Klärfilters, die Zimtsäure durch Ansäuern mit Salz- oder Schwefelsäure in hervorragender Qualität kristallin ausfällen und abscheiden. Nach dieser Verfahrensvariante erhältliche Alkali- oder Ammoniumcinnamatlösungen sind aufgrund ihrer Reinheit auch ohne Zwischenisolierung der freien Säure zu weiteren Umsetzungen, z.B. zur Herstellung von Phenylalanin zu verwenden.

Die nach dem erfindungsgemässen Verfahren hergestellten gegebenenfalls substituierten Zimtsäuren liegen in der trans-Form vor.

Nach dem erfindungsgemässen Verfahren erhält man Zimtsäuren in hohen Ausbeuten und grosser Reinheit. Überraschenderweise bilden sich Nebenprodukte wie Styrol nur in sehr geringen Mengen.

Zimtsäure und ihre kernsubstituierten Derivate sind technisch wichtige Ausgangsmaterialien zur Herstellung biologischer und pharmazeutischer Wirkstoffe, von Süssstoffen, optischen Aufhellern, Flüssigkristallen und Zwischenprodukten für zahlreiche Zwecke (Ullmanns Encyclopädie der technischen Chemie, 4. Auflage, Band 24, Seite 592).

*Beispiel 1*

In einer Rührapparetur werden 106 g Benzaldehyd unter Stickstoffatmosphäre mit 2 g Zinkacetat-dihydrat und 1,5 ml Essigsäure in 150 ml Toluol gelöst. In diese Lösung leitet man bei 30 bis 40°C innerhalb von 3 bis 4 Stunden Ketengas ein, das im Laboratoriumsmasstab leicht durch thermische Spaltung von Diketen erzeugt werden kann (vgl. Houben-Weyl, Band VII/4, Seite 80; Org. Syntheses, Coll. Vol. V, p. 679). Nach Umsetzung von 92 bis 96% des Benzaldehyds, ermittelt durch gaschromatographische Analyse, unterbricht man die Ketenzufuhr, rührt das Reaktionsgemisch noch kurze Zeit nach und überführt es in einen Niederdruck-Autoklaven aus VA-Stahl. Man gibt 4 g 92 %iges Natriumhydroxid-Pulver hinzu und destilliert Toluol und überschüssigen Benzaldehyd am absteigenden Kühler in eine Vorlage ab. Darauf erhitzt man den viskosen, hellen Rückstand 1 Stunde lang auf 180°C, nimmt das erhaltene Reaktionsprodukt nach dem Abkühlen auf ca. 100°C in 700 ml Wasser auf und bringt es durch Zugabe von 140 ml 20%igem Ammoniak in Lösung. Nach Zusatz von ca. 20 g Aktiv-Kohle rührt man noch kurze Zeit bei 60 bis 70°C, klärfiltriert und säuert das Filtrat mit 30%iger Salzsäure bis zur deutlich sauren Reaktion an. Dabei fällt Zimtsäure in weissen Kristallen aus, die abgesaugt, mit wenig angesäuertem kaltem Wasser gewaschen und bei 70°C im Trockenschrank getrocknet werden.

Ausbeute: 109 g farblose kristalline Zimtsäure vom F. 133 bis 134°C; unter Berücksichtigung von 8,5 g wiedereinsetzbarem Benzaldehyd im Toluol-Destillat (gaschromatographisch ermittelt) beträgt die Ausbeute 80,2% d. Th.

Verwendet man anstelle von Zinkacetat die gleichmolare Menge Zinkisobutyrat oder Zinkpivalat oder Zinkpropionat, so erhält man bei gleich hervorragender Produktqualität ähnlich hohe Ausbeuten.

*Beispiel 2*

Eine unter Stickstoffatmosphäre hergestellte Lösung von 106 g Benzaldehyd in 1 20 ml Dichlorbenzol versetzt man mit 2 g Zinkacetat-dihydrat und 2 ml Eisessig. Unter Rühren wird Ketengas eingeleitet, wobei man die Temperatur von ca. 20 bis 40°C steigen lässt. Nach dem Umsatz von ca. 94% des Benzaldehyds (nach gaschromatographischer Bestimmung) rührt man noch 30 Minuten bei 40°C nach.

Das Einleiten des Ketens dauert etwa 4 Stunden; bei Aussenkühlung lässt sich die Reaktionsdauer auf etwa die Hälfte vermindern. Darauf gibt man 4 g Natriumhydroxid-Pulver hinzu und erhitzt auf 180°C. Nach halbstündigem Rühren bei dieser Temperatur lässt man auf 100°C abkühlen und 700 ml Wasser, sowie 140 ml konzentrierten wässrigen Ammoniak zufliessen. Man rührt das Gemisch eine halbe Stunde bei 60°C, lässt die Phasen absitzen, trennt die (untere) Dichlorbenzol-Phase ab und klärfiltriert die wässrige Phase unter Verwendung von 10 g Aktiv-Kohle. Das erhaltene wasserhelle Filtrat wird angesäuert und die ausgefallene kristalline Zimtsäure wird abgesaugt, mit wenig kaltem Wasser gewaschen und im Trockenschrank bei 70°C getrocknet.

Ausbeute: 120,2 g; weisse Kristalle vom F. 133 bis 134°C; Gehalt 99,5% = 86% d. Th., unter Berücksichtigung von 6,3 g wiedergewonnemen Benzaldehyd. Die Chlorbenzolphase kann nach Ersatz des verbrauchten Benzaldehyds und Trocknung erneut in die Keten-Anlagerung eingesetzt werden. Verwendet man anstelle des Zinkacetats die gleiche Menge Eisenacetat, so erhält man Zimtsäure mit ähnlich guter Produktqualität in einer Ausbeute von 79% d. Th.

Ähnlich hohe Ausbeuten (84 bis 86% d. Th.) erzielt man mit Zinkpropionat, Zinkbutyrat, Zinkisobutyrat, Zinkpivalat und Zinkisovalerat anstelle des Zinkacetats.

Mit Zinkstearat, Zinkbenzoat, Zink-hexahydrobenzoat und Zinkcinnamat als Katalysatoren werden Zimtsäure-Ausbeuten von 76 bis 80% d. Th. erhalten.

*Beispiel 3*

Verfährt man nach den Angaben des Beispiels 2, setzt jedoch bei der Nachbehandlung des Primärproduktes anstelle von Natriumhydroxid die folgenden basischen bzw. sauren Katalysatoren ein, so erhält man die in der Tabelle aufgeführten, nicht optimierten Ausbeuten an Zimtsäure:

| Katalysator | Ausbeute (% d. Th.) | Katalysatormenge (g) |
|---|---|---|
| a Kaliumhydroxid-Pulver | 84 | 4 |
| b Calciumhydroxid-Pulver | 62 | 5 |
| c Natriumacetat, wasserfrei | 77 | 5 |
| d 4-Dimethylaminopyridin | 83 | 3 |
| e Tributylamin | 68 | 4 |
| f Diazabicyclo-nonen | 84 | 4 |
| g Diazabicyclo-undecen | 82 | 4 |
| h Schwefelsäure | 78 | 6 |
| i Chlorwasserstoffgas | 80 | 8 |
| k Toluolsulfonsäure | 64 | 5 |

*Beispiel 4*

140 g 4-Chlorbenzaldehyd werden zusammen mit 2 g Zinkacetat und 2 ml Essigsäure in 100 ml Dichlorbenzol gelöst. Bei 30 bis 40°C wird bis zu einem Umsatz von 90% des 4-Chlorbenzaldehyds Ketengas eingeleitet. Darauf gibt man 3 g Natriumhydroxidpulver hinzu und erhitzt 1 Stunde auf 180°C, lässt auf 120°C abkühlen und giesst das Gemisch unter Rühren in 600 ml Wasser und 120 ml konzentrierten Ammoniak. Nach halbstündigem Nachrühren trennt man die wässrige Phase von der Dichlorbenzolphase ab, klärt unter Zusatz von 20 g Aktivkohle und säuert das klare Filtrat mit Salzsäure an. Die in weissen Kristallen ausgefallene 4-Chlorzimtsäure saugt man ab, wäscht sie mit wenig kaltem Wasser und trocknet sie.

In der Dichlorbenzolphase sind gemäss gaschromatographischer Bestimmung 12 g wiederverwendbarer 4-Chlorbenzaldehyd enthalten.

Ausbeute: 121 g 4-Chlor-Zimtsäure, farblose Kristalle F. 248 bis 250°C; = 72,5% d. Th., bezogen auf umgesetzten 4-Chlorbenzaldehyd.

In entsprechender Weise sind die folgenden substituierten Zimtsäuren aus den betreffenden Aldehyden mit den jeweils in der Tabelle angegebenen Schmelzpunkten erhältlich.

| Beispiel | Zimtsäurederivat | F. |
|---|---|---|
| 5 | 4-Methyl-zimtsäure | 197-198° |
| 6 | 2-Fluor-zimtsäure | 176-178° |
| 7 | 4-Methoxy-zimtsäure | 172/191° |
| 8 | 3-Nitro-zimtsäure | 200-202° |
| 9 | 4-Phenyl-zimtsäure | 222° |
| 10 | 3,4-Methylendioxyzimtsäure | 238° |
| 11 | 2-Chlor-5-nitro-zimtsäure | 219-220° |
| 12 | 2,4-Dichlor-zimtsäure | 229-231° |
| 13 | 4-Brom-zimtsäure | 249-251° |

**Patentansprüche**

1. Verfahren zur Herstellung von gegebenenfalls substituierten Zimtsäuren durch Umsetzung des entsprechenden Benzaldehyds mit Keten in Gegenwart eines Katalysators, dadurch gekennzeichnet. dass man einen Katalysator aus der Gruppe der Eisen- und/oder Zinksalze einer Mono- oder Dicarbonsäure mit 2 bis 20 Kohlenstoffatomen, wobei die einzusetzenden Katalysatoren einen Anteil der entsprechenden freien Carbonsäuren von 1 - 80 Gew.-%, bezogen auf den gesamten Katalysator enthalten, verwendet und die Umsetzung in einem Lösungsmittel durchführt und dann das Reaktionsprodukt in Gegenwart eines sauren oder basischen Katalysators im Temperaturbereich von 100 bis 250°C behandelt.

2. Verfahren nach Anspruch 1. dadurch gekennzeichnet, dass für die Eisen- und/oder Zinksalze Mono- oder Dicarbonsäuren der Formel

$$X - A - COOH$$

in der

X Carboxyl oder Wasserstoff bedeutet,

A einen zweibindigen Rest, der sich aus der Gruppe gesättigter oder ungesättigter aliphatischer Kohlenwasserstoffe mit 1 bis 18 Kohlenstoffatomen, cycloaliphatischer Kohlenwasserstoffe mit 5 bis 12 Kohlenstoffatomen, araliphatischer Kohlenwasserstoffe mit 7 bis 78 Kohlenstoffatomen oder aromatischer Kohlenwasserstoffe mit 6 bis 1 2 Kohlenstoffatomen ableitet, und für den Fall, dass X für eine Carboxylgruppe steht, auch eine Einfachbindung bedeuten kann,

verwendet werden.

3. Verfahren nach den Ansprüchen 1 und 2, dadurch gekennzeichnet, dass man 0,1 bis 10 Mol-% des Katalysators, bezogen auf den gegebenenfalls substituierten Benzaldehyd einsetzt.

4. Verfahren nach den Ansprüchen 1 bis 3, dadurch gekennzeichnet, dass die Umsetzung mit Keten im Temperaturbereich von -10 bis + 100°C durchgeführt wird.

5. Verfahren nach den Ansprüchen 1 bis 4, dadurch gekennzeichnet, dass man als basischen Katalysator eine Alkali- oder Erdalkaliverbindung, ein Amin oder eine Azaverbindung einsetzt.

6. Verfahren nach den Ansprüchen 1 bis 4, dadurch gekennzeichnet, dass man als sauren Katalysator eine starke Säure einsetzt.

7. Verfahren nach den Ansprüchen 1 bis 6, dadurch gekennzeichnet, dass man 0.05 bis 0.20 Mol des sauren

oder basischen Katalysators, bezogen auf 1 Mol des eingesetzten gegebenenfalls substituierten Benzaldehyds verwendet.

## Claims

1. Process for the preparation of optionally substituted cinnamic acids by reaction of the corresponding benzaldehyde with ketene in the presence of a catalyst, characterized in that a catalyst from the group comprising iron and/or zinc salts of a mono- or dicarboxylic acid having 2 to 20 carbon atoms is used, the catalysts to be used containing an amount of the corresponding free carboxylic acids of 1-80% by weight, based on the entire catalyst, and the reaction being carried out in a solvent and the reaction product then being treated in the temperature range from 100 to 250°C in the presence of an acid or basic catalyst.

2. Process according to Claim 1, characterized in that for the iron and/or zinc salts mono- or dicarboxylic acids of the formula

   $$X - A - COOH$$

   in which
   X denotes carboxyl or hydrogen and
   A can denote a divalent radical which is derived from the group comprising saturated or unsaturated aliphatic hydrocarbons with 1 to 18 carbon atoms, cycloaliphatic hydrocarbons with 5 to 12 carbon atoms, araliphatic hydrocarbons with 7 to 18 carbon atoms or aromatic hydrocarbons with 6 to 12 carbon atoms and, if X represents a carboxyl group, also a single bond,
   are used.

3. Process according to Claims 1 and 2, characterized in that 0.1 to 10 mol % of the catalyst, based on the optionally substituted benzaldehyde, is employed.

4. Process according to Claims 1 to 3, characterized in that the reaction with ketene is carried out in a temperature range from -10 to +100°C.

5. Process according to Claims 1 to 4, characterized in that an alkali metal compound, an alkaline earth metal compound, an amine or an aza compound is employed as the basic catalyst.

6. Process according to Claims 1 to 4, characterized in that a strong acid is employed as the acid catalyst.

7. Process according to Claims 1 to 6, characterized in that 0.05 to 0.20 mol of the acid or basic catalyst per mole of the optionally substituted benzaldehyde employed is used.

## Revendications

1. Procédé de préparation d'acides cinnamiques éventuellement substitués par réaction du benzaldéhyde correspondant avec le cétène en présence d'un catalyseur, caractérisé en ce que l'on utilise un catalyseur du groupe des sels de fer et/ou de zinc d'un acide mono- ou dicarboxylique en $C_2$-$C_{20}$, les catalyseurs à utiliser contenant une proportion des acides carboxyliques libres correspondants de 1 à 80 % en poids, par rapport au catalyseur total, et l'on conduit la réaction dans un solvant puis on traite le produit de réaction en présence d'un catalyseur acide ou basique dans l'intervalle de températures de 100 à 250°C.

2. Procédé selon la revendication 1, caractérisé en ce que l'on utilise des sels de fer et/ou de zinc d'acides mono- ou dicarboxyliques de formule:

   $$X - A - COOH$$

   dans laquelle
   X représente le groupe carboxyle ou l'hydrogène,
   A représente un groupe divalent dérivant d'hydrocarbures aliphatiques saturés ou insaturés en $C_1$-$C_{18}$, d'hydrocarbures cycloaliphatiques en $C_5$-$C_{12}$, d'hydrocarbures araliphatiques en $C_7$-$C_{18}$ ou d'hydrocarbures aromatiques en $C_6$-$C_{12}$, et peut également représenter une liaison simple dans le cas où X représente un groupe carboxyle.

3. Procédé selon les revendications 1 et 2, caractérisé en ce que l'on utilise de 0,1 à 10 mol % du catalyseur

par rapport au benzaldéhyde éventuellement substitué.

4. Procédé selon les revendications 1 à 3, caractérisé en ce que la réaction avec le cétène est effectuée dans un intervalle de températures de -10 à +100°C.

5. Procédé selon les revendications 1 à 4, caractérisé en ce que l'on utilise en tant que catalyseur basique un composé alcalin ou alcalino-terreux, une amine ou un composé en aza.

6. Procédé selon les revendications 1 à 4, caractérisé en ce que l'on utilise en tant que catalyseur acide un acide fort.

7. Procédé selon les revendications 1 à 6, caractérisé en ce que l'on utilise de 0,05 à 0,20 mol du catalyseur acide ou basique pour 1 mol du benzaldéhyde éventuellement substitué mis en oeuvre.